# EUROPEAN PATENT APPLICATION

(11) **EP 2 915 556 A1**
(43) Date of publication of application: **09.09.2015**
(21) Application number: 15162634.8
(22) Date of filing: 20.01.2011
(51) Int. Cl.: A61M 25/10, A61M 25/00

(54) **BALLOON CATHETERS WITH THERAPEUTIC AGENT IN BALLOON FOLDS AND METHODS OF MAKING THE SAME**

(30) Priority: 21.01.2010 US 296991 P
(62) Divisional of application: 11703071.8
(71) Applicant: Boston Scientific Scimed, Inc., Maple Grove, MN 55311 (US)
(72) Inventor: Kangas, Steve, Woodbury, MN Minnesota 55125 (US)
(74) Representative: Peterreins, Frank

(57) **Abstract**

Catheter devices for delivering therapeutic agent and methods of making the same. A method comprises applying, when the balloon is unexpanded and folded, a first coating to a first surface area of the balloon that is substantially exposed when the balloon is unexpanded and folded, the first coating being a coating to which the therapeutic agent coating material is substantially non-adherent; applying a therapeutic agent coating material to the balloon such that the therapeutic agent coating material adheres to a second surface area not coated with the first coating. Upon completion of the coating method, the therapeutic agent coating is substantially disposed within one or more folds of the balloon. A catheter device comprises therapeutic agent coating that is substantially covered when the balloon is unexpanded and folded. Expanding the balloon opens the one or more folds and substantially exposes the therapeutic agent.

## Description

### CROSS REFERENCE TO RELATED APPLICATION

The present application claims priority to United States provisional application Serial No. 61/296,991 filed January 21, 2010, the disclosure of which is incorporated herein by reference in its entirety.

### TECHNICAL FIELD

The present invention relates to medical devices, more particularly to catheter devices.

### BACKGROUND

Catheters are used in a wide variety of minimally-invasive or percutaneous medical procedures. For example, balloon catheters having drug coatings may be used to treat diseased target tissue, such as portions of blood vessels. Typically, for treatment of blood vessels, the balloon is inserted through a peripheral blood vessel and then guided via a catheter through the vascular system to the target intravascular site. However, as the balloon travels through the body, the flow of blood may wash away some of the drug coating, or the drug coating may otherwise become detached. This not only can result in an undesired loss of drug, but it can also result in drug being supplied to undesired parts of the body. Therefore, there is a need for improved catheter-based devices for drug delivery to a target site.

### SUMMARY

In one embodiment, the present disclosure provides a method of manufacturing a medical device comprising providing a catheter with a balloon mounted on the catheter, the balloon having one or more folds when the balloon is in an unexpanded and folded state, wherein the one or more folds open upon expansion of the balloon; providing a first coating formed from a first coating material and a second coating formed from a second coating material, the first coating being a coating to which the second coating material is substantially non-adherent; when the balloon is in the unexpanded and folded state, applying the first coating material to form a first coating on a first surface area of the balloon that is substantially exposed when the balloon is in the unexpanded and folded state; applying the second coating material to form a second coating on a second surface area of the balloon that is not coated with the first coating, such that the second coating is substantially disposed within the one or more folds. The second coating may be applied to the balloon when the balloon is in an expanded state. Alternatively, the second coating may be applied to the balloon when the balloon is in an unexpanded and folded state. The second coating may be disposed only within the one or more folds when the balloon is in the unexpanded and folded state. The second coating may comprise a therapeutic agent.

In another embodiment, the present disclosure provides a medical device comprising a catheter; a balloon mounted on the catheter, the balloon having one or more folds when the balloon is in an unexpanded and folded state, wherein the one or more folds open upon expansion of the balloon; a first coating formed from a first coating material and a second coating formed from a second coating material; wherein the first coating is a coating to which the second coating material is substantially non-adherent; wherein the first coating is located on a first surface area of the balloon that is substantially exposed when the balloon is in the unexpanded and folded state; wherein the second coating is located on a second surface area of the balloon that is substantially covered when the balloon is in the unexpanded and folded state, such that the second coating is substantially disposed within the one or more folds when the balloon is in the unexpanded and folded state; and wherein expanding the balloon opens the one or more folds and substantially exposes the second coating. The second coating may comprise a therapeutic agent such that expanding the balloon exposes the second coating for delivery of the therapeutic agent to a target site.

In another embodiment, the present disclosure provides a method of treating a target location in a body with therapeutic agent, the method comprising: using a medical device comprising a catheter; a balloon mounted on the catheter, the balloon having one or more folds when the balloon is in an unexpanded and folded state, wherein the one or more folds open upon expansion of the balloon; a first coating formed from a first coating material; and a second coating formed from a second coating material; wherein the first coating is a coating to which the second coating material is substantially non-adherent; wherein the first coating is located on a first surface area of the balloon that is substantially exposed when the balloon is in the unexpanded and folded state; wherein the second coating is located on a second surface area of the balloon that is substantially covered when the balloon is in the unexpanded and folded state, such that the second coating is substantially disposed within the one or more folds when the balloon is in the unexpanded and folded state; delivering the balloon in the unexpanded and folded state to the target location; and expanding the balloon at the target location, thereby opening the one or more folds and substantially exposing the second coating. The second coating may comprise a therapeutic agent such that expanding the balloon exposes the second coating for delivery of the therapeutic agent to a target site.

### BRIEF DESCRIPTION OF THE DRAWINGS

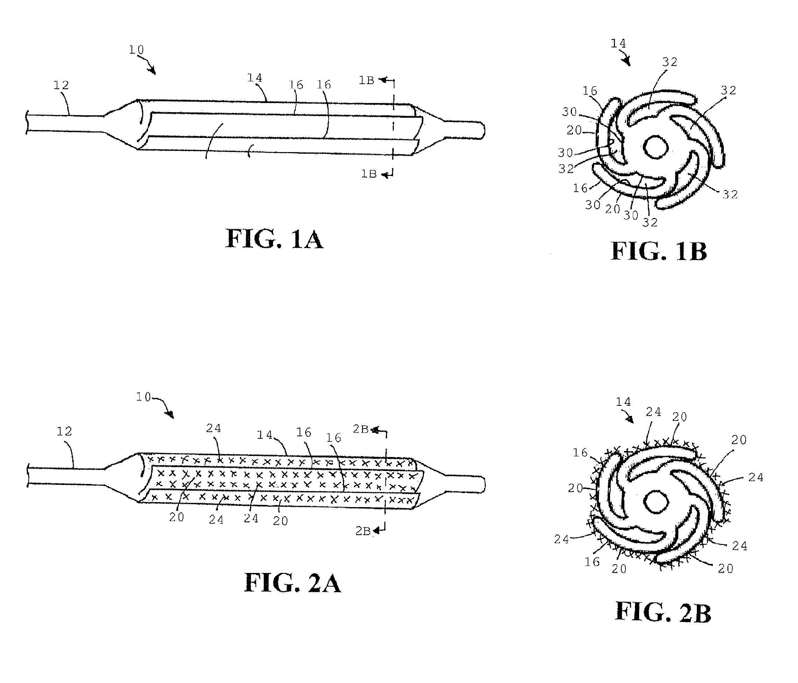

FIGS. 1A-1B show a catheter device to be coated according to an embodiment of the present disclosure. FIG. 1A shows the catheter device with the balloon in an unexpanded and folded state. FIG. 1B shows an enlarged, cross-sectional view of the balloon of FIG. 1A.
FIGS. 2A-2B show a first step in a method of manufacturing a catheter device according to an embodiment of the present disclosure. FIG. 2A shows the catheter device of FIG. 1A, still with the balloon in an unexpanded and folded state, with a first coating applied to surface area of the balloon that is substantially exposed when the balloon is in the unexpanded and folded state. FIG. 2B shows an enlarged, cross-sectional view of the balloon of FIG. 2A.

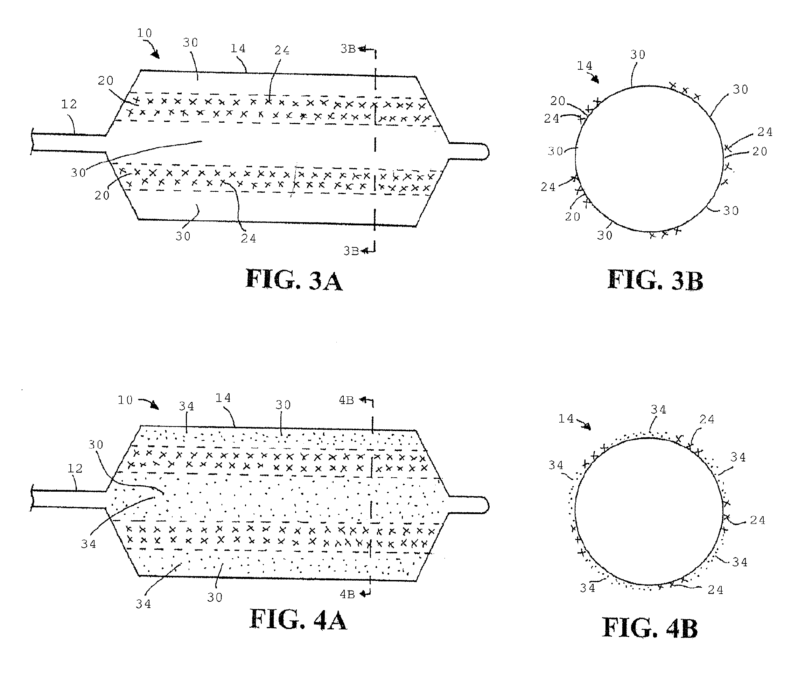

FIGS. 3A-3B show a subsequent step in a method of manufacturing a catheter device according to an embodiment of the present disclosure. FIG. 3A shows the catheter device of FIG. 2A in an expanded state. FIG. 3B shows a cross-sectional view of the balloon of FIG. 3A.
FIGS. 4A-4B show a subsequent step in a method of manufacturing a catheter device according to an embodiment of the present disclosure. FIG. 4A shows the catheter device of FIG. 3A, with the balloon still in an expanded state, with a second coating material applied to the balloon such that the second coating adheres to surface area of the balloon that is substantially covered when the balloon is in the unexpanded and folded state. FIG. 4B shows a cross-sectional view of the balloon of FIG. 4A.

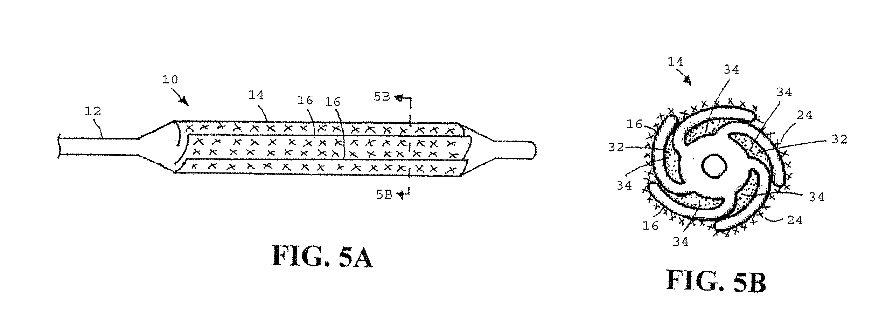

FIGS. 5A-5B show a subsequent step in a method of manufacturing a catheter device according to an embodiment of the present disclosure. FIG. 5A shows the catheter device with the balloon returned to the unexpanded and folded state. FIG. 5B shows an enlarged, cross-sectional view of the balloon of FIG. 5A.

### DETAILED DESCRIPTION

Catheter devices of the present disclosure use an expandable balloon for delivering a therapeutic agent to a target site in the body. The balloon is designed to be insertable into the body via a catheter. The therapeutic agent can be associated with the balloon in any of various ways, as further described below. Any of various mechanisms conventionally used for the delivery, actuation, or expansion (*e.g*., by inflation) of balloon catheter devices may be used in the present invention. The balloon catheter may be designed similar to those that have been known in the art, including but not limited to angioplasty catheters, stent delivery catheters, inflation catheters, and/or perfusion catheters. The catheter devices of the present disclosure may be used in conjunction with other drug delivery devices, such as stents.

The balloon has one or more folds which serve as reservoirs for containing a therapeutic agent or drug (the terms "therapeutic agent" and "drug" are used interchangeably herein). The folds may be oriented in any of various ways on the balloon, including, but not limited to, for example, longitudinally, radially, circumferentially, or helically. The folds may be made by any of the methods known in the art, including but not limited to methods described in U.S. Patent/Publication Nos. 5,342,307 (Enteneuer et al.), 5,147,302 (Enteneuer et al.), 5,458,572 (Campbell et al.), 5,954,740 (Ravenscroft et al.), 6,013,055 (Bampos et al.), 7,128,868 (Eidenschink), 7,306,616 (Eidenschink et al.), or 2004/0215227 (McMorrow et al.), all of which are incorporated herein by reference.

Examples of folds, and the ways in which the folds may hold therapeutic agent, are disclosed in U.S. Patent Application Publication No. 2009/0227949 A1 (Knapp et al.), the disclosure of which is also incorporated herein by reference in its entirety.

The folds may have any of various configurations to hold the therapeutic agent. For example, the folds may be in the form of pockets, grooves, dimples or wells. The folds are not limited to structures formed by the bending, creasing or pleating of the balloon wall. Folds may also be formed as voids within the balloon wall itself *(e.g.,* as grooves, channels, or trenches), which may be made during extrusion or by etching, stamping, laser ablation or heat ablation of the balloon.

Because the folds of the balloon serve as reservoirs for containing the therapeutic agent, the therapeutic agent is substantially covered when the balloon is in the unexpanded, deflated state. As the balloon is expanded, *e.g.,* by inflation, the folds open such that the therapeutic agent is exposed and allowed to be delivered to the target location.

Since the drug is typically intended to be delivered only to the target site, it would generally be considered undesirable for drug to come off of the balloon while the balloon is being tracked to the target site, for example through the vasculature to a coronary artery. Analysis of some current drug-coated balloons has shown significant particulate generation during tracking. This indicates that drug is coming off of the balloon or otherwise being released before intended, which can lead not only to undesired loss of drug but also to drug being delivered to places other than to the intended target site. In a folded balloon coated with drug, the majority of particulates generated during tracking originates from coating on the outside of the folds of the balloon, *i.e.,* from coating on surface area of the balloon that is exposed to the outside when the balloon is in a deflated, folded state. Methods have been proposed to load drug only in the folds, or on surface area of the balloon that is not exposed to the outside when the balloon is in a deflated, folded state. Such methods include, for example, microsyringe coating and methods disclosed in U.S. Patent Application Publication No. 2009/0227949 A1. However, with at least some of these methods, the process still can result in coating the balloon outside of the folds. In addition, at least some of these methods can be time consuming, complicated and/or expensive.

Accordingly, the present disclosure provides an advantageous method of applying a therapeutic agent coating to a balloon such that the therapeutic agent coating adheres, primarily or exclusively, to surface area of the balloon that is substantially covered when the balloon is in the folded state. When the balloon is in the folded state, the therapeutic agent is disposed within one or more folds of the balloon, thereby protecting it from release or detachment during tracking.

FIGS. 1A-1B show a catheter device 10 to be coated according to an embodiment of the present disclosure. FIG. 1A shows the catheter device 10 with a balloon 14 in an unexpanded state, in which the balloon 14 is deflated and folded. FIG. 1B shows an enlarged, cross-sectional view of the balloon 14 of FIG. 1A. As can be seen in FIG. 1A, the catheter device 10 comprises a balloon 14 mounted on an elongate shaft 12. Balloon 14 in this embodiment is folded into a series of lobes in the form of pleats 16, in a manner known in the art as discussed above. The outside surface of each pleat 16 comprises a portion of the surface area 20 of the balloon 14 that is exposed to the outside when the balloon 14 is in the unexpanded and folded state. Underneath each pleat 16 is a fold in the form of a folded pocket 32 comprising surface area 30 of the balloon that is covered and thus not exposed when the balloon 14 is in the unexpanded and folded state.

FIGS. 2A-2B show a first step in a method of coating the catheter device 10 according to an embodiment of the present disclosure. FIG. 2A shows the catheter device 10 of FIG. 1A, with the balloon 14 still in an unexpanded and folded state, with a first coating material applied to form a first coating 24 on the surface area 20 of the balloon that is exposed when the balloon 14 is in the unexpanded and folded state. FIG. 2B shows an enlarged, cross-sectional view of the balloon 14 of FIG. 2A. The first coating 24 is a coating that will adhere to the balloon 14; however, the first coating 24 is a coating to which a subsequently applied second coating material is substantially non-adherent. In this context, a second coating material is "substantially non-adherent" to a first coating when: either (i) upon application of the second coating material, the second coating material substantially does not adhere to the first coating but does adhere to areas of the balloon not covered by the first coating, such that none or only a relatively minimal amount of the second coating adheres to the first coating, or (ii) after application of the second coating material, the second coating material is more easily removed from the first coating than from areas of the balloon not covered by the first coating by utilizing a subsequent process *(e.g.,* a peeling process, solvent application or other process), so as to leave none or only a relatively minimal amount of the second coating material adhering to the first coating. The first coating 24 may be a coating that is substantially non-wettable by a subsequently applied second coating material. In this context, "substantially non-wettable" means that upon application of the second coating material, the second coating material substantially does not wet the first coating and thus does not adhere to the first coating, but the second coating material does adhere to areas of the balloon not covered by the first coating. When the first coating is substantially non-wettable by the second coating material, the first coating will also be a coating to which the second coating material is substantially non-adherent.

Because the balloon 14 is in an unexpanded state, with the folds 32 substantially covered, when the first coating 24 is applied, the first coating 24 is selectively coated onto the balloon 14, contacting and adhering only to the surface area 20 of the balloon 14 that is exposed when the balloon 14 is in the unexpanded and folded state. The composition of the first coating 24 can be any of a number of suitable coatings that can adhere to the balloon 14, or to a coating on the balloon 14, but to which the subsequently-applied second coating material is substantially non-adherent. For example, the first coating 24 may be a Teflon copolymer, for example poly(4,5-difluoro-2,2-bis(trifluoromethyl)-1,3-dioxole-co-tetrafluoroethylene), which is soluble in, for example, Fluorinert™ FC-72 (3M Co.). Other examples of substances that may be applied as the first coating 24 include other fluoropolymer copolymers such as polyfluoroethylene-co-vinyl ether and fluoropolymers described in "Fluoronated Coating and Finishing Handbook" (Laurence McKeen), fluoroacrylate polymers such as Novec™ (3M Co.) fluoropolymers, and silicone polymers such as polymers and copolymers of polydimethylsiloxane.

The first coating material forming the first coating 24 may be applied to the balloon 14 in a number of suitable ways such that the first coating adheres to the exposed surface area 20 of the unexpanded and folded balloon 14. For example, the first coating material may be applied to the balloon 14 by sponge-coating the first coating material onto the unexpanded and folded balloon 14. In this way, the first coating 24 is applied only to the outside surfaces of the pleats 16. Other methods are, of course, possible, such as spray coating, brush coating, roller coating, dip coating, syringe coating or other methods that apply the first coating 24 to the exposed surface area 20 of the unexpanded and folded balloon 14.

FIGS. 3A-3B show a subsequent step in the method of coating the catheter device 10. In this step, the balloon 14 is expanded, either fully or partially, such that the surface area 30 to be coated with the second coating 34 is substantially exposed. FIG. 3A shows the catheter device 10 of FIG. 2A in this expanded, substantially unfolded state. FIG. 3B shows a cross-sectional view of the balloon 14 of FIG. 3A. As can be seen in FIGS. 3A-3B, the first coating 24 is adhered to surface area 20 of the balloon that had been exposed when the balloon 14 was unexpanded and folded. When the balloon is in the expanded state (e.g., partially or fully inflated), the surface area 30 that was covered when the balloon 14 was unexpanded and folded is now exposed.

FIGS. 4A-4B show a subsequent step in the method of coating the catheter device 10. FIG. 4A shows the catheter device 10 of FIG. 3A, still with the balloon 14 in an expanded state, with a second coating material applied to the balloon 14 to form a second coating 34 on the surface area 30 of the balloon 14 that will be substantially covered when the balloon 14 is returned to the unexpanded and folded state. FIG. 4B shows a cross-sectional view of the balloon 14 of FIG. 4A. In this embodiment, the second coating 34 comprises a therapeutic agent desired to be delivered to a target site.

The composition of the second coating material to form the second coating 34 can be any of a number of suitable coating materials that will be substantially non-adherent to the first coating 24 but that will adhere to the surface area 30 of the balloon 14 (or a coating on the balloon 14) where the balloon 14 (or a coating on the balloon 14) is not covered by the first coating 24. For example, the second coating material to form the second coating 34 may comprise paclitaxel in a solution of tetrahydrofuran (THF) and ethanol (EtOH). When applied to a first coating comprising poly(4,5-difluoro-2,2-bis(trifluoromethyl)-1,3-dioxole-co-tetrafluoroethylene), such a second coating material will be substantially non-wetting and will be substantially non-adherent to the second coating. Other examples of solvents that may be used in the application of the second coating include isopropyl alcohol, ketones, toluene, ethers, hydrocarbon solvents, water and blends of the above solvents.

The second coating material to form the second coating 34 may be applied to the balloon 14 in a number of suitable ways. For example, the balloon 14 may be dip-coated into a coating solution. Other methods are, of course, possible, such as spray coating, brush coating, roller coating, sponge coating, syringe coating or other methods that apply the second coating material to form the second coating 34 on the balloon 14.

Because the second coating material is substantially non-adherent to the first coating 24, none or only a relatively minimal amount of the second coating 34 adheres to the first coating 24 in the completed product. In some embodiments, the second coating material is in the form of a solution that substantially does not wet out on the first coating 24 and thus substantially does not adhere to the first coating 24 upon application. In other embodiments, the second coating material may initially adhere to the first coating 24 to some degree, but the second coating material is more easily removed from the first coating 24 than from areas of the balloon 14 not covered by the first coating 24 by utilizing a subsequent process *(e.g.,* a peeling process, solvent application or other suitable process). For example, the second coating material, after drying, may be peeled off of the first coating 24 or removed by another suitable mechanical process. As another example, a solvent that removes the second coating material from the first coating 24 but not from other areas may be applied. In this way, the subsequent process leaves none or only a relatively minimal amount of the second coating 34 adhering to the first coating 24. In either case, whether the second coating material initially does not adhere or is later removed, substantially all of the second coating 34 in the completed product adheres only to the surface area 30 of the balloon 14 (or a coating on the balloon 14) where the balloon 14 is not covered by the first coating 24. Substantially no second coating 34 adheres to the surface area 20 of the balloon that is exposed when the balloon 14 is unexpanded and folded. Thus, the second coating 34 is substantially covered when the balloon 14 is returned to the unexpanded and folded state.

FIGS. 5A-5B show a subsequent step in the method of coating the catheter device 10. After the second coating 34 has been applied, the balloon 14 is returned to its unexpanded and folded state. FIG. 5A shows the catheter device with the balloon 14 returned to its unexpanded and folded state. FIG. 5B shows an enlarged, cross-sectional view of the balloon 14 of FIG. 5A. As can be seen in FIGS. 5A-5B, the second coating 34 on surface area 30 of the balloon 14 is substantially covered when the balloon 14 is in the unexpanded and folded state. In this way, the second coating is substantially disposed within the folds 32.

The catheter device 10 is used in a manner substantially similar to the use of drug coated catheters as disclosed in U.S. Patent Application Publication No. 2009/0227949 A1 and other drug coated balloon catheters. The distal end of the catheter device 10 is inserted into the patient and the balloon 14 is tracked, for example through the vasculature, to the target site. Because the second coating 34 comprising the therapeutic agent is substantially covered and not exposed when the balloon 14 is in its unexpanded and folded stated for delivery, the therapeutic agent is protected while balloon 14 is being guided to the target site. In this way, loss of drug from the balloon 14 during tracking is substantially avoided.

When the balloon 14 is at the target site, for example at a target location within a coronary artery, the balloon 14 is expanded by inflation. Upon expansion of the balloon 14, the balloon 14 is returned to the state shown in FIGS. 4A-4B. The folds 18 open during expansion. Opening of the folds 18 substantially exposes the second coating 34, thereby exposing the therapeutic agent for delivery to the vessel. As the second coating 34 contacts or is pressed against the area to be treated, the drug is transferred and thereby delivered to the area to be treated.

Various alternative embodiments to the embodiment illustrated in FIGS. 1A-5B are possible. For example, instead of expanding the balloon to apply the therapeutic agent coating, the therapeutic agent can be applied to the unexpanded balloon after the first coating has been applied, by injecting the therapeutic agent coating into the folds by a syringe-coating process. In this way, the drug coating would be applied and would adhere only within the folds.

Other alternatives to the illustrated embodiment are also possible. For example, as described above, the folds may be oriented longitudinally as illustrated, or in alternative ways, for example radially, circumferentially, or helically. The width, pitch, pitch angle and depth of the folds can vary depending upon the particular application. There may be any suitable number and arrangement of folds. The folds may be in the form of pockets as shown, or, for example, as grooves, dimples, wells, channels or trenches. The edges of the folds that cooperate to form a compartment for the therapeutic agent may touch or be close together without touching. For example, the edge of a pleat 16 in FIGS. 5A-5B may touch the adjacent balloon surface or be close to it. If desired, the edges may be held together, for example, by an adhesive, laser welding, heat setting, biodegradable or bioerodable sutures or stitching, or some other means by which the edges may be held closely together or touching during balloon delivery but which will allow the edges to separate upon balloon expansion. For example, after the deflating step in FIGS. 5A-5B, the edges of the pleats 16 may be adhered to the adjacent balloon surfaces to close the compartments of therapeutic agent. Upon inflation, the inflation force overcomes the adhesion such that the pleat 16 is separated from the rest of the balloon 14, thereby allowing the balloon 14 to expand.

A catheter according to the present disclosure may include a vascular stent mounted on the balloon. The vascular stent may be any suitable stent known in the art, including those with or without coatings that elute a therapeutic agent. The stent may also be biostable, bioerodable or biodegradable.

The balloons of the present invention may also be coated with other compounds that can aid in the transfer of the therapeutic agent, such as sugars, oils, fatty acids, surfactants, water soluble or dispersible oligomers and polymers, and plasticizers such as citrate esters, for example, acetyltributyl citrate. These compounds may be a separate coating or may be blended with the therapeutic agent. The balloons of the present invention also may be coated with a radiocontrast agent (ionic or non-ionic), such as iopromide. The contrast agent may be a separate coating or may be blended with the therapeutic agent. Example

A 2% (wt/wt) solution ofpoly(4,5-difluoro-2,2-bis(trifluoromethyl)-1,3-dioxole-co-tetrafluoroethylene) in Fluorinert™ FC-72 (3M Co.) is prepared. A small sponge is saturated with the coating solution. The sponge is wiped over the surface of an unexpanded, folded Liberte® balloon. The catheter is dried in a convection oven at 50°C. The balloon is then inflated to about 1 atm. and dip-coated in a 20% solution of paclitaxel in THF/EtOH (40/60) and dried. The paclitaxel coating only wets out and coats the regions of the balloon not coated by the first coating (the balloon surface that was the interior of the folds). The balloon is then refolded, resulting in a balloon with drug only within the folds.

The therapeutic agent used with embodiments of the disclosure may be any suitable pharmaceutically acceptable agent (such as a drug), a biomolecule, a small molecule or cells. Exemplary drugs include anti-proliferative agents such as paclitaxel, sirolimus (rapamycin), tacrolimus, everolimus, biolimus, and zotarolimus. Exemplary biomolecules include peptides, polypeptides and proteins; antibodies; oligonucleotides; nucleic acids such as double- or single-stranded DNA (including naked and cDNA), RNA, antisense nucleic acids such as antisense DNA and RNA, small interfering RNA (siRNA), and ribozymes; genes; carbohydrates; angiogenic factors including growth factors; cell cycle inhibitors; and anti-restenosis agents. Exemplary small molecules include hormones, nucleotides, amino acids, sugars, and lipids and compounds having a molecular weight of less than 100kD. Exemplary cells include stem cells, progenitor cells, endothelial cells, adult cardiomyocytes, and smooth muscle cells. Further therapeutic agents that may be used with embodiments of the disclosure are disclosed in U.S. Patent Application/Publication Nos. 2005/0037050 (Weber), 61/074,456, 61/185,745 and 61/267,944, the disclosures of which are incorporated herein by reference.

The foregoing description and embodiments are not intended to be limiting. Each of the disclosed aspects and embodiments may be considered individually or in combination with other aspects, embodiments, and variations. Modifications of the disclosed embodiments incorporating the spirit and substance of the invention may occur to persons skilled in the art within the scope of the present invention.

### Embodiments

1. A method of manufacturing a medical device comprising:
   providing a catheter with a balloon mounted on the catheter, the balloon having one or more folds when the balloon is in an unexpanded and folded state, wherein the one or more folds open upon expansion of the balloon;
   when the balloon is in the unexpanded and folded state, applying a first coating material to form a first coating on a first surface area of the balloon that is substantially exposed when the balloon is in the unexpanded and folded state, the first coating being a coating to which a second coating material is substantially non-adherent; and
   applying the second coating material to form a second coating on a second surface area of the balloon that is not coated with the first coating, such that the second coating is substantially disposed within the one or more folds when the balloon is in the unexpanded and folded.
2. The method of embodiment 1, wherein the second coating comprises a therapeutic agent.
3. The method of embodiment 1, further comprising the steps of:
   expanding the balloon prior to applying the second coating material;
   applying the second coating material to the balloon when the balloon is in an expanded state; and
   returning the balloon to the unexpanded and folded state after applying the second coating material to the balloon, such that the second coating is substantially disposed within the one or more folds when the balloon is in the unexpanded and folded state.
4. The method of embodiment 1, wherein the step of applying the second coating material is performed while the balloon is in the unexpanded and folded state.
5. The method of embodiment 1, wherein the balloon assumes a substantially cylindrical shape when the balloon is fully expanded, and wherein the second coating is disposed on the outer surface of the cylindrical-shaped balloon when the balloon is fully expanded.
6. The method of embodiment 1, wherein the second coating is disposed only within the one or more folds when the balloon is in the unexpanded and folded state.
7. The method of embodiment 1, wherein the first coating comprises a copolymer of tetrafluoroethylene.
8. The method of embodiment 1, wherein the first coating material is applied to the balloon by sponge coating.
9. The method of embodiment 1, wherein the first coating material is applied to the balloon by dip coating.
10. The method of embodiment 1, wherein the first coating material is applied to the balloon by a syringe.
11. The method of embodiment 1, wherein the second coating material is applied to the balloon by sponge coating.
12. The method of embodiment 1, wherein the second coating material is applied to the balloon by dip coating.
13. The method of embodiment 1, wherein the second coating material is applied to the balloon by syringe.
14. The method of embodiment 1, wherein the first coating is substantially non-wettable by the second coating material.
15. A medical device comprising:
   a catheter;
   a balloon mounted on the catheter, the balloon having one or more folds when the balloon is in an unexpanded and folded state, wherein the one or more folds open upon expansion of the balloon;
   a first coating formed from a first coating material; and
   a second coating formed from a second coating material;
   wherein the first coating is a coating to which the second coating material is substantially non-adherent;
   wherein the first coating is located on a first surface area of the balloon that is substantially exposed when the balloon is in the unexpanded and folded state;
   wherein the second coating is located on a second surface area of the balloon that is substantially covered when the balloon is in the unexpanded and folded state, such that the second coating is substantially disposed within the one or more folds when the balloon is in the unexpanded and folded state; and
   wherein expanding the balloon opens the one or more folds and substantially exposes the second coating.
16. The medical device of embodiment 15, wherein the second coating comprises a therapeutic agent, and wherein expanding the balloon opens the one or more folds and substantially exposes the second coating for delivery of the therapeutic agent to a target site.
17. The medical device of embodiment 15, wherein the balloon assumes a substantially cylindrical shape when the balloon is fully expanded, and wherein the second coating is disposed on the outer surface of the cylindrical-shaped balloon when the balloon is fully expanded.
18. The medical device of embodiment 15, wherein the second coating is disposed only within the one or more folds when the balloon is in the unexpanded and folded state.
19. A method of treating a target location in a body with therapeutic agent, the method comprising:
   using a medical device comprising:
      a catheter;
      a balloon mounted on the catheter, the balloon having one or more folds when the balloon is in an unexpanded and folded state, wherein the one or more folds open upon expansion of the balloon;
      a first coating formed from a first coating material; and
      a second coating formed from a second coating material, the second coating comprising a therapeutic agent;
      wherein the first coating is a coating to which the second coating material is substantially non-adherent;
      wherein the first coating is located on a first surface area of the balloon that is substantially exposed when the balloon is in the unexpanded and folded state; and
      wherein the second coating is located on a second surface area of the balloon that is substantially covered when the balloon is in the unexpanded and folded state, such that the therapeutic agent is substantially disposed within the one or more folds when the balloon is in the unexpanded and folded state;
      delivering the balloon in the unexpanded and folded state to the target location; and
      expanding the balloon at the target location, thereby opening the one or more folds and substantially exposing the therapeutic agent for delivery to the target location.
20. The method of embodiment 19, wherein the second coating is disposed only within the one ormore folds when the balloon is in the unexpanded and folded state.

## Claims

1. A medical device comprising:
a catheter;
a balloon mounted on the catheter, the balloon having one or more folds when the balloon is in an unexpanded and folded state, wherein the one or more folds open upon expansion of the balloon;
a first coating formed from a first coating material; and
a second coating formed from a second coating material;
wherein the first coating is a coating to which the second coating material is substantially non-adherent;
wherein the first coating is located on a first surface area of the balloon that is substantially exposed when the balloon is in the unexpanded and folded state;
wherein the second coating is located on a second surface area of the balloon that is substantially covered when the balloon is in the unexpanded and folded state,
such that the second coating is substantially disposed within the one or more folds when the balloon is in the unexpanded and folded state; and
wherein expanding the balloon opens the one or more folds and substantially exposes the second coating.

2. The medical device of claim 1, wherein the second coating comprises a therapeutic agent, and wherein expanding the balloon opens the one or more folds and substantially exposes the second coating for delivery of the therapeutic agent to a target site.

3. The medical device of claim 1, wherein the balloon assumes a substantially cylindrical shape when the balloon is fully expanded, and wherein the second coating is disposed on the outer surface of the cylindrical-shaped balloon when the balloon is fully expanded.

4. The medical device of claim 1, wherein the second coating is disposed only within the one or more folds when the balloon is in the unexpanded and folded state.

5. The medical device of claim 1, wherein the balloon assumes a substantially cylindrical shape when the balloon is fully expanded, and wherein the second coating is disposed on the outer surface of the cylindrical-shaped balloon when the balloon is fully expanded.

6. The medical device of claim 1, wherein the second coating is disposed only within the one or more folds when the balloon is in the unexpanded and folded state.

7. The medical device of claim 1, wherein the first coating comprises a copolymer of tetrafluoroethylene.

8. The medical device of claim 1, wherein the first coating is substantially non-wettable by the second coating material.
